# EUROPÄISCHE PATENTSCHRIFT

(11) **EP 0 529 420 B1**
(45) Veröffentlichungstag und Bekanntmachung des Hinweises auf die Patenterteilung: **26.03.1997**
(21) Anmeldenummer: 92113787.3
(22) Anmeldetag: 13.08.1992
(51) Int. Cl.: G01N 11/04, G01N 33/48, G01N 33/49, F04B 43/08

(54) **Vorrichtung für die Untersuchung von Fliessvorgängen in einer transparenten Kapillare**
Device for investigating flow processes in a transparent capillary
Dispositif pour l'examen de l'écoulement dans un capillaire transparent

(30) Priorität: 29.08.1991 DE 4128695; 29.08.1991 DE 4128696
(43) Veröffentlichungstag der Anmeldung: 03.03.1993
(73) Patentinhaber: MYRENNE GmbH SPEZIALMASCHINEN + APPARATEBAU, D-52159 Roetgen (DE)
(72) Erfinder: Myrenne, Heinz, W-5106 Roetgen (DE); Schmid-Schönbein, Holger, Prof. Dr. med., W-5102 Würselen Broichweiden (DE)
(74) Vertreter: Biermann, Wilhelm, Dr.-Ing.

(56) Entgegenhaltungen:
- EP-A- 0 013 155
- DD-A- 153 434
- DE-A- 2 842 737
- DE-A- 3 338 364
- US-A- 3 463 614

## Beschreibung

Die Erfindung betrifft eine Vorrichtung für die Untersuchung von Fließvorgängen mit einer lichtdurchlässigen Kapillare, einer optischen Beobachtungseinrichtung und einer Pumpe für die Erzeugung eines Volumenstroms der zu untersuchenden Flüssigkeit in der Kapillare.

Eine Vorrichtung dieser Art ist aus der DE 3338364 A1 bekannt. Sie dient zur Durchführung mikrorheologischer Untersuchungen von Teilchensuspensionen, insbesondere zur Messung der Eigenschaften der individuellen Viskoelastizität und des Fließverhaltens von deformierbaren Zellen wie Erythrozyten. Die Pumpe saugt die zu untersuchende Flüssigkeit aus einem Vorratsbehälter an und fördert sie in das Innere des Kapillarrohrs.

Zur Durchführung mikrorheologischer Untersuchungen innerhalb einer transparenten Kapillare muß die zu untersuchende Flüssigkeit mit Hilfe der Pumpe mit einem definierten Volumenstrom durch die Kapillare gepumpt werden. Je nach der Viskosität der zu untersuchenden Flüssigkeit, dem Strömungsdurchmesser der Kapillare und der gewünschten Strömungsgeschwindigkeit innerhalb der Kapillare müssen unter Umständen hohe Drücke aufgebracht werden.

üblicherweise werden als Pumpen für die Erzeugung der Flüssigkeitsströmung in Vorrichtungen zur Durchführung mikrorheologischer Untersuchungen in einer transparenten Kapillare Kolbenpumpen eingesetzt. Kolbenpumpen benötigen zum Lösen des Kolbens aus der Ruhestellung eine größere Kraft als während des nachfolgenden Bewegungsvorgangs, was zu Unregelmäßigkeiten zu Beginn des Strömungsvorgangs führt. Diese unvermeidliche Erscheinung, die auch als Slip-Stick-Phänomen bezeichnet wird, wirkt sich für exakte Messungen störend aus.

Der Erfindung liegt die Aufgabe zugrunde, eine Vorrichtung für die Durchführung mikrorheologischer Untersuchungen bereitzustellen, mit der innerhalb der Meßkapillare hohe Drücke und definierte störungsfreie Volumenströme erzeugt werden können.

Gemäß der Erfindung wird diese Aufgabe dadurch gelöst, daß die Pumpe ein mit einer inkompressiblen Flüssigkeit gefülltes einseitig geschlossenes Rohr ist, das an eine Verformungseinrichtung angekoppelt ist und mit deren Hilfe einer definierten elastischen Biegung untertworfen wird, unter deren Wirkung sich das flüssigkeitsgefüllte Innenvolumen ändert.

In zweckmäßiger Weiterbildung der Erfindung ist das federelastisch verformbare Rohr in der Art eines umgekehrt geschalteten Röhrenfedermanometers kreisförmig gebogen, beispielsweise in Form eines Zweidrittelkreises, und weist einen elliptischen oder abgeflachten Querschnitt auf.

Daß ein kreisförmig gebogenes Federrohr seine Form verändert, wenn sich der Druck innerhalb des Rohres ändert, ist bekannt. Von diesem Effekt wird bei dem sogenannten Röhrenfedermanometer Gebrauch gemacht, indem man die Deformation des gebogenen Federrohres bei Luftdruckänderungen über geeignete mechanische oder elektrische Verstärkungsmittel sichtbar macht. Bei der erfindungsgemäßen Vorrichtung wird dieses Prinzip umgekehrt, indem auf das Federrohr eine definierte elastische Verformung aufgebracht wird, die eine Änderung des Innenvolumens des Federrohres zur Folge hat. Durch Füllung des Innenvolumens des Federrohres mit einer inkompressiblen Flüssigkeit wird durch die Volumenänderung des Federrohres die Flüssigkeit um ein entsprechendes Maß verdrängt und erzeugt in der hiermit in Verbindung stehenden Kapillare den gewünschten Volumenstrom.

Mit Hilfe der erfindungsgemäßen Vorrichtung lassen sich kurzzeitige in einer Richtung verlaufende Strömungen in der Kapillare erzeugen, solange das Federrohr in ein und derselben Richtung gebogen wird.

Gemäß einer besonders vorteilhaften Weiterbildung der erfindungsgemäßen Vorrichtung ist das elastisch verformbare Federrohr an eine mechanische Verformungseinrichtung angekoppelt, die eine zyklische Biegung und Rückbiegung des Federrohres erzeugt. Auf diese Weise wird eine oszillierende Pumpe geschaffen, durch die die Flüssigkeitssäule in der Beobachtungskapillare in eine schwingende Strömung versetzt wird.

Der Weg des Volumenstroms in der Kapillare und die Oszillationsfrequenz lassen sich dabei durch entsprechende Dimensionierung und konstruktive Ausgestaltung des Federrohres und der Verformungseinrichtung in weiten Grenzen ändern. Beispielsweise kann der Antriebsmotor für den oszillierenden Dehnungs- und Entdehnungsvorgang des Federrohres aus einem regelbaren Getriebemotor bestehen, wodurch die Oszillationsfrequenz gesteuert wird. Zur Einstellung des Volumenstroms kann beispielsweise das Element zur Kraftübertragung vom Antriebsmotor auf das Federrohr aus einem konischen Exzenterkörper bestehen, so daß durch Verschieben des Exzenterkörpers entlang seiner Drehachse der auf das Federrohr einwirkende Hub veränderbar ist.

In weiterer vorteilhafter Ausgestaltung der Erfindung weist die lichtdurchlässige Kapillare einen flachen Strömungsquerschnitt von 0,5 bis 2 mm Breite unds 0,05 bis 0,2 mm Höhe auf. Eine für den erfindungsgemäßen Zweck besonders zweckmäßige Glaskapillare hat einen Strömungsquerschnitt von etwa 1 mm Breite und etwa 0,1 mm Höhe.

Vorrichtungen mit der erfindungsgemäß verwendeten Pumpe und einer stark abgeflachten Kapillare lassen sich grundsätzlich für die Untersuchung der verschiedensten Fließvorgänge der unterschiedlichsten Teilchensuspensionen einsetzen. Besondere Vorteile hat die Verwendung einer derartigen Vorrichtung bei der Bestimmung der Aggregationsgeschwindigkeit von Erythrozyten in einer Blutprobe, indem durch gezielte Strömung innerhalb einer lichtdurchlässigen Meßzelle in der Blutprobe vorhandene Erythrozytenaggregationen aufgelöst und nach plötzlicher Unterbrechung der Strömung der Aggregationsvorgang in der Meßzelle beobachtet wird. So werden beispielsweise in einer erfindungsgemäß eingesetzten Kapillare sehr hohe Schergrade (über 5000/s) und daher ein sehr gutes Dispersionsvermögen erreicht. Der Schergrad, die entscheidende Größe für die Dispersion der Erythrozytenaggregate, hängt vom Unterschied der Strömungsgeschwindigkeiten benachbarter Flüssigkeitsschichten ab. Da die Schichten mit minimaler Strömungsgeschwindigkeit unmittelbar an den Kapillarwänden und die Schichten mit maximaler Strömungsgeschwindigkeit in der zentralen Zone der abgeflachten Kapillare sehr dicht beieinander liegen, ergibt sich nämlich in diesem Fall ein sehr hoher Strömungsgeschwindigkeitsgradient. Die Auflösung der Erythrozytenaggregate in einer solchen Flachkapillare ist deshalb besonders wirkungsvoll, so daß hiermit auch die schwersten Ausprägungsgrade von pathologischen Erythrozytenaggregationen hydrodynamisch dispergiert und damit vor der darauffolgenden Messung aufgelöst werden. Die vollständige hydrodynamische Dispersion der Erythrozytenaggregationen läßt sich durch die Wände der abgeflachten Kapillare hindurch beispielsweise Laser-diffraktometrisch nachweisen.

Die Flachkapillare ermöglicht darüber hinaus in optimaler Weise die optische Auswertung, denn innerhalb der durchstrahlten Meßfläche findet keine störende Ablenkung der Lichtstrahlen statt, wie sie beispielsweise bei Kapillaren mit rundem Querschnitt durch die dioptrische Wirkung der gekrümmten Glaswände erfolgt. Bei der Untersuchung der Aggregatbildung in Suspensionen läßt sich sowohl während der Dispersionsphase als auch während der Aggregationsphase durch Ausblenden der Randzonen der Flachkapillare die Untersuchung der Vorgänge in der Strömung auf das Mittelfeld der Flachkapillare beschränken, in dem verhältnismäßig einfache und überschaubare Strömungsverhältnisse herrschen.

Bei der Untersuchung des Fließ- und Aggregationsverhaltens von Erythrozyten in Blutproben hat es sich ferner gezeigt, daß Meßungenauigkeiten, die bei bekannten Verfahren dadurch bedingt sind, daß bei diesen nicht nur das Ausmaß der Erythrozytenaggregation, sondern auch die Konzentration der Blutzellen die Lichttransmission bestimmt, beim erfindungsgemäßen Verfahren weitgehend ausgeschaltet werden. Bei den bekannten Verfahren besteht in der dort vorhandenen sogenannten viskosimetrischen Strömung zwischen der Abnahme der optischen Dichte, die durch die Aggregation der Erythrozyten bedingt ist, und der Abnahme der optischen Dichte, die durch die Verringerung der Zellkonzentration bedingt ist, eine unstete Beziehung mit einer deutlichen Spitze bei der biologisch wichtigen Zellkonzentration von 35 Volumen-%. überraschenderweise ergibt sich demgegenüber bei dem erfindungsgemäßen Verfahren über den gesamten biologisch relevanten Bereich von Erythrozytenkonzentrationen, das heißt im Bereich zwischen 10 % und 55 % Volumenanteile Erythrozyten im Plasma, ein linearer Zusammenhang zwischen dem jeweiligen Aggregationssignal und dem Volumenanteil der Erythrozyten. Dadurch werden die Auswertung und die Korrektur der Meßergebnisse stark vereinfacht. Aufgrund dieser empirisch festgestellten Tatsache ist das praktische Vorgehen bei Routine-Untersuchungen deutlich einfacher als bei den bekannten Verfahren. Während nämlich bei den bekannten Verfahren bei Präzisionsmessungen der Zellvolumenanteil sorgfältig eingestellt werden muß, kann mit dem erfindungsgemäßen Verfahren das zu untersuchende Blut, dessen Zusammensetzung zunächst immer unbekannt ist, unmittelbar ohne Vorbehandlung untersucht werden. Das Meßergebnis wird dann später nach der Bestimmung des Zellvolumenanteils rechnerisch korrigiert. Diese Tatsache erlaubt es ferner, auf einen Stabilisator für das Blut zur Gerinnungshemmung entweder ganz zu verzichten oder einen solchen Stabilisator zu verwenden, der das Blut in seiner natürlichen Zusammensetzung beläßt.

Die Verwendung von Meßzellen mit stark abgeflachtem Querschnitt erlaubt ferner den Einsatz von modernen Beleuchtungseinrichtungen wie lichtemittierenden Dioden und von modernen lichtelektrischen Wandlern wie Fotodioden, so daß die Beleuchtungs- und die Meßeinrichtung in miniaturisierter Form und mit optimalem Wirkungsgrad ausgebildet werden können.

Weitere Merkmale und Vorteile der Erfindung ergeben sich aus den Unteransprüchen und aus der nachfolgenden Beschreibung eines bevorzugten Ausführungsbeispiels einer erfindungsgemäßen Vorrichtung anhand der Zeichnungen.

Von den Zeichnungen zeigt
- Fig. 1: eine erfindungsgemäße Vorrichtung mit ihren wesentlichen Teilen in einer schematischen Darstellung;
- Fig. 2: den Querschnitt einer Meßzelle in stark vergrößerter Darstellung, und
- Fig. 3: eine bevorzugte Ausführungsform einer Oszillationspumpe.

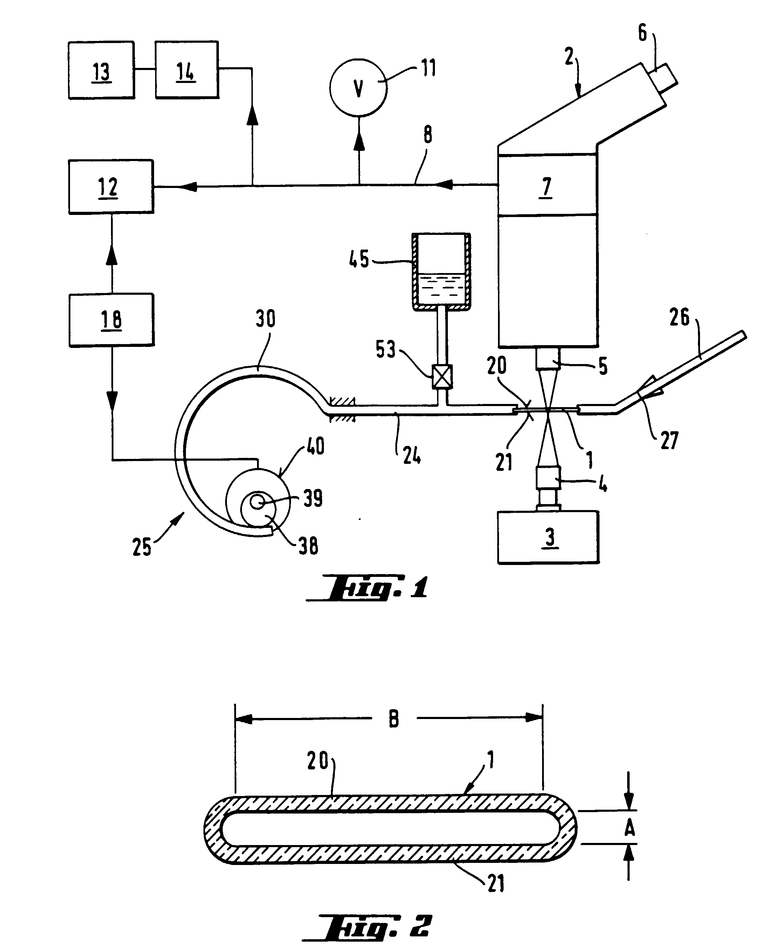

Wie aus der schematischen Darstellung in Fig. 1 hervorgeht, ist die als Meßzelle dienende Glaskapillare 1 im Strahlengang eines Durchlichtmikroskops 2 angeordnet. Für eine konstante Beleuchtung sorgen die Lichtquelle 3 und die Kondensorlinse 4. Das Objektiv 5 des Mikroskops weist beispielsweise einen Vergrößerungsfaktor von V=40 auf, während das Okular 6 einen Vergrößerungsfaktor von V=10 aufweist. Das Mikroskop 2 ist mit einem Fotometeraufsatz 7 versehen. Kern des Fotometeraufsatzes 7 ist ein Fotovervielfacher, der den vom Objektiv 5 erfaßten Lichtstrom proportional in ein rauscharmes Gleichspannungssignal guter Langzeitkonstanz umwandelt. Dieses Gleichspannungssignal wird über die Leitung 8 den Meß- und Auswerteeinheiten zugeführt.

Zu den Meß- und Auswerteeinheiten gehören ein Spannungsmesser 11, ein Hochgeschwindigkeitsschreiber 12 und ein Mikrocomputer 13 mit vorgeschaltetem Analog-Digital-Wandler 14. Der Spannungsmesser 11 dient zur Information und Kontrolle des absoluten Spannungssignals, insbesondere während der Kalibrierung der Meßvorrichtung. Der Hochgeschwindigkeitsschreiber 12 zeichnet die der Transmissionsänderung während der Aggregationsphase entsprechende Spannungsänderung auf. Der Mikrocomputer 13 schließlich dient zur Speicherung und Auswertung des Meßsignals. Die Meßvorrichtung umfaßt ferner eine elektronische Steuereinheit 18.

Die als Meßzelle dienende Glaskapillare 1 weist den in Fig. 2 dargestellten Querschnitt auf. Sie hat zwei planparallele Wände 20, 21, deren gegenseitiger Abstand A etwa 0,1 mm beträgt. Die Breite B der Glaskapillare 1 beträgt etwa 1 mm und ihre Länge einige Zentimeter.

Die Glaskapillare 1 ist auf der einen Seite über eine druckfeste Leitung 24 mit einer Oszillationspume 25, und auf der anderen Seite über eine Steckverbindung 27 mit einem Kapillarrohr 26 verbunden, das die zu untersuchende Blutprobe enthält.

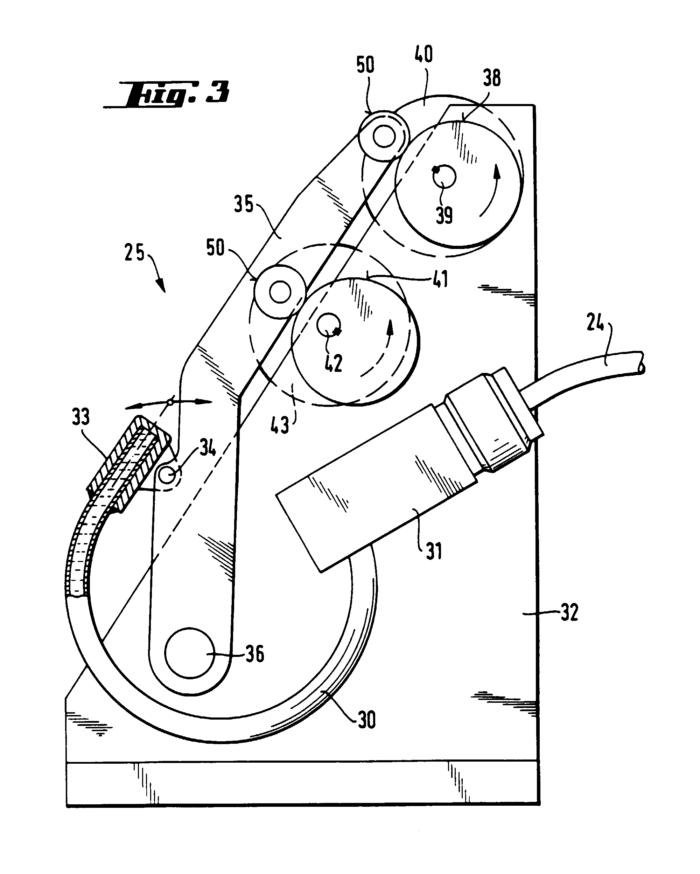

Die Oszillationspumpe 25 besteht, wie im einzelnen in Fig. 3 dargestellt ist, aus einem einseitig geschlossenen elastisch verformbaren Federrohr 30 mit elliptischem Querschnitt, das zu einem Zweidrittel-Kreis gebogen ist, wie es für sogenannte Röhrenfeder-Manometer verwendet wird. Das eine Ende des Federrohres 30 ist über ein Kupplungsstück 31 an einer Grundplatte 32 festgelegt und mit der druckfesten Leitung 24 verbunden. Das andere Ende des Federrohres 30 ist von einer Hülse 33 umgeben, die einen Zapfen 34 trägt. An dem Zapfen 34 greift ein Hebel 35 an, der seinerseits an der Grundplatte 32 über ein Drehlager 36 schwenkbar gelagert ist. Mit Hilfe des Hebels 35 wird das Federrohr 30 elastisch aufgebogen, wodurch sich das Innenvolumen des Federrohres 30 geringfügig vergrößert. Das elastische Aufbiegen des Federrohres 30 erfolgt mit Hilfe einer Kurvenscheibe 38, die von einem Getriebemotor 40 mit der gewünschten Drehzahl in Drehung versetzt wird. Die Kurvenscheibe 38 ist im dargestellten Fall eine kreisförmige Scheibe, die exzentrisch auf der Welle 39 des auf der anderen Seite der Grundplatte 32 angeordneten Getriebemotors 40 befestigt ist. Der die Kurvenscheibe 38 in Drehung versetzende Getriebemotor 40 ist so ausgelegt, daß die elastische Verformung des Federrohres 30 auf eine Verformungsfrequenz von 0,1 bis 3 pro Sekunde einstellbar ist.

Das Innenvolumen des Federrohres 30 ist mit einer inkompressiblen Flüssigkeit gefüllt, die sich nicht mit den anderen Flüssigkeiten in dem System vermischt, beispielsweise mit einem Fluorchlorkohlenwasserstoff. Die sich anschließende druckfeste Leitung 24 ist mit einer ebenfalls inkompressiblen Koppelflüssigkeit gefüllt, das heißt mit einer mit der zu untersuchenden Flüssigkeit kompatiblen Flüssigkeit. Diese Flüssigkeitssäule überträgt die durch die Verformung des Federrohres 30 bedingte Volumenverschiebung auf die zu untersuchende Flüssigkeit in der Glaskapillare 1 und bewirkt dort die oszillatorische Strömung.

Die Oszillationspumpe 25 ist mit einer zweiten Kurvenscheibe 41 versehen. Diese Kurvenscheibe 41 ist an der Grundplatte 32 in geringerer Entfernung zum Drehlager 36 des Hebels 35 angeordnet als die Kurvenscheibe 38, so daß durch diese Kurvenscheibe 41 der Hebel 35 weiter ausgelenkt und damit das Federrohr 30 um ein größeres Maß aufgebogen wird. Die Kurvenscheibe 41, bei der es sich im dargestellten Fall wieder um eine exzentrisch gelagerte kreisförmige Scheibe handelt, ist außerdem mit einer größeren Exzentrizität auf der Welle 42 des auf der anderen Seite der Grundplatte 32 angeordneten Getriebemotors 43 befestigt. Die größere Verformung des Federrohres 30 bewirkt eine größere Volumenänderung und dadurch einen größeren Volumenstrom innerhalb des Kapillarrohres 24 und wird zum Füllen und zum Entleeren der Glaskapillare 1 benutzt. Die Kurvenscheiben 38, 41 können, um die Reibung zu reduzieren, über Rollen 50 auf den Hebel 35 wirken.

Die die Oszillationspumpe 25 mit der Glaskapillare 1 verbindende druckfeste Leitung 24 steht über ein Ventil 53 mit einem Behälter 45 in Verbindung, der die Koppelflüssigkeit enthält, mit der die druckfeste Leitung 24 gefüllt ist.

Mit Hilfe dieser Vorrichtung soll beispielsweise die Aggregationsgeschwindigkeit der Erythrozyten in einer Blutprobe bestimmt werden. In diesem Fall wird als Koppelflüssigkeit für die Füllung der druckfesten Leitung 24 beispielsweise eine 1 %-ige Natriumchloridlösung oder die sogenannte Ringerlösung verwendet. Um die Meßkapillare 1 mit der zu untersuchenden Blutprobe zu füllen, wird das Kapillarrohr 26 mit der Blutprobe in die Steckverbindung 27 eingesteckt und die Blutprobe bei geschlossenem Ventil 53 von der Oszillationspumpe 25 bis hinter die Meßkapillare 1 eingesogen. Dabei wird das Federrohr 30 von der Kurvenscheibe 41 aufgedehnt, deren Hub auf die Füllmenge des Systems abgestimmt ist. Anschließend wird das Ventil 53 geöffnet, um die Koppelflüssigkeit, die sich zwischen der zu untersuchenden Blutprobe und dem Pumpfluid befindet, beim Zurückbiegen des Federrohres 30 in die Zuleitung zum Behälter 45 zu pumpen und dort zwischenzulagern. Dabei bleibt das Füllvolumen in der Meßkapillare 1 aufgrund des mehr als zehnfach geringeren Querschnitts der Meßkapillare gegenüber dem Querschnitt der Zuleitung 24 und der viermal so hohen Viskosität des Blutes gegenüber der Viskosität der Koppelflüssigkeit unverändert. Nun wird das Ventil 53 geschlossen, und der oszillative Volumenfluß zur Dispersion der Blutprobe in der Kapillare 1 kann beginnen, indem die Kurvenscheibe 38 das Federrohr 30 rhythmisch auf- und zubiegt, wobei das Volumen in der Kapillare 1 oszilliert. Nach 5 bis 10 Sekunden wird die Pumpe schnell gestoppt, wobei die "Härte" des Pumpsystems den Stillstand der Meßflüssigkeit innerhalb weniger Millisekunden gewährleistet. Nun beginnt die Lichttransmissionsmessung durch das Fotometer 7.

Nach der Messung wird zur Entfernung der Blutprobe das Kapillarrohr 26 entfernt und verworfen (Einmalartikel), das Ventil 53 geöffnet und die in der Zuleitung zum Behälter 45 zwischengespeicherte Koppelflüssigkeit von der Oszillationspumpe aufgenommen. Jetzt schließt das Ventil 53, und die Pumpe 25 drückt die untersuchte Flüssigkeit und die Koppelflüssigkeit durch die Kapillare 1 und die Steckverbindung 27 heraus in ein Entsorgungsgefäß. Dabei wird die Pumpe 25 wieder über die Kurvenscheibe 41 mit dem größeren Hub angetrieben. Durch wiederholtes Ansaugen von Koppelflüssigkeit aus dem Behälter 45 und Ausdrücken durch die Kapillare 1 wird das System gespült und steht danach für eine erneute Probenaufnahme zur Verfügung.

Es hat sich gezeigt, daß bei der beschriebenen Geometrie der Glaskapillare 1 eine gute Auflösung der Erythrozytenaggregationen erreicht wird, wenn die Oszillationsfrequenz der Pumpe 0,5 bis 1,5 pro Sekunde beträgt. Bei einem Fördervolumen der Oszillationspumpe von etwa 100 Mikrolitern pro Hub werden in einer Glaskapillare mit den beschriebenen Abmessungen in der Mittelebene Strömungsgeschwindigkeiten von 0,6 bis 2 m/s erreicht. Die hierdurch bewirkten Schergrade in dem strömenden Blut (über 3000/s) sind so hoch, daß innerhalb einer verhältnismäßig kurzen Zeit von nur 1 Sekunde eine vollständige Auflösung der Erythrozytenaggregationen erfolgt.

Als lichtelektrische Wandler für die Transmissionsmessung können anstelle des Mikroskop-Fotometers, wie es bei dem beschriebenen Ausführungsbeispiel benutzt wird, eine oder mehrere Fotodioden eingesetzt werden, die unmittelbar oberhalb der Glaskapillare angeordnet werden. Durch die Verwendung von lichtemittierenden, auf die Wellenlänge der Fotodioden abgestimmten Dioden als Lichtquelle lassen sich die Abmessungen der Vorrichtung stark verkleinern. Insbesondere läßt sich das von einer LED ausgesendete Lichtstrahlenbündel durch Blenden mit sehr kleinem Durchmesser von etwa 0,2 mm so stark bündeln, daß hierdurch nur der mittlere Bereich der Glaskapillare durchstrahlt wird, in dem verhältnismäßig einfache Strömungsverhältnisse herrschen, so daß die Genauigkeit der Messungen hierdurch weiter gesteigert werden kann.

## Patentansprüche

1. Vorrichtung für die Untersuchung von Fließvorgängen mit einer lichtdurchlässigen Kapillare (1), einer optischen Beobachtungseinrichtung (2) und einer Pumpe (25) für die Erzeugung eines Volumenstroms der zu untersuchenden Flüssigkeit in der Kapillare, **dadurch gekennzeichnet,** daß die Pumpe (25) ein mit einer inkompressiblen Flüssigkeit gefülltes federelastisch verformbares einseitig geschlossenes Rohr (30) ist, das an eine Verformungseinrichtung (38,39,40) angekoppelt ist und mit deren Hilfe einer definierten elastischen Biegung unterworfen wird, unter deren Wirkung sich das flüssigkeitsgefüllte Innenvolumen ändert.

2. Vorrichtung nach Anspruch 1, dadurch gekennzeichnet, daß das federelastisch verformbare Rohr (30) in der Art eines umgekehrt geschalteten Röhrenfedermanometers kreisförmig gebogen ist und einen elliptischen oder abgeflachten Querschnitt aufweist.

3. Vorrichtung nach Anspruch 1 oder 2, dadurch gekennzeichnet, daß die an das elastisch verformbare Federrohr (30) angekoppelte Verformungsvorrichtung (38,39,40), eine zyklische Biegung und Rückbiegung des Federrohres (30) erzeugt.

4. Vorrichtung nach Anspruch 3, dadurch gekennzeichnet, daß als Verformungseinrichtung (38,39,40) für das Federrohr (30) eine von einem Getriebemotor (40) angetriebene Kurvenscheibe (38) dient.

5. Vorrichtung nach Anspruch 4, dadurch gekennzeichnet, daß zur Veränderung der Amplitude des Volumenstroms die Kurvenscheibe (38) als konischer Exzenterkörper ausgebildet und auf der angetriebenen Drehachse verschiebbar angeordnet ist.

6. Vorrichtung nach Anspruch 4 oder 5, dadurch gekennzeichnet, daß die Form der Kurvenscheibe (38) oder des Exzenterkörpers so gestaltet ist, daß zwischen den beiden Umkehrpunkten der Biegebewegung ein konstanter Volumenstrom erzeugt wird.

7. Vorrichtung nach einem oder mehreren der Ansprüche 1 bis 6, dadurch gekennzeichnet, daß die Oszillationsfrequenz des Federrohres (30) von 0,1 bis 3,0 Hz einstellbar ist.

8. Vorrichtung nach einem oder mehreren der Ansprüche 1 bis 7, dadurch gekennzeichnet, daß das Federrohr (30) an eine zweite Kurvenscheibe (41) ankoppelbar ist, die von einem eigenen Getriebemotor (43) angetrieben ist und dem Federrohr (30) einen größeren Dehnungshub erteilt als die die oszillierende Pumpwirkung erzeugende Kurvenscheibe (38).

9. Vorrichtung nach einem oder mehreren der Ansprüche 1 bis 8, dadurch gekennzeichnet, daß die lichtdurchlässige Kapillare (1) eine flachen Strömungsquerschnitt von 0,5 bis 2 mm Breite und 0,05 bis 0,2 mm Höhe aufweist.

10. Vorrichtung nach einem oder mehreren der Ansprüche 1 bis 9, dadurch gekennzeichnet, daß die optische Beobachtungseinrichtung (2) eine Beleuchtungseinrichtung mit einer Lichtquelle (3) und ein die die Kapillare (1) durchdringende Lichtmenge erfassendes Mikroskop-Fotometer (7) umfaßt.

11. Vorrichtung nach einem oder mehreren der Ansprüche 1 bis 9, dadurch gekennzeichnet, daß die optische Beobachtungseinrichtung eine Beleuchtungseinrichtung mit einer Lichtquelle (3) und eine oder mehrere die die Kapillare durchdringende Lichtmenge erfassende Fotodioden umfaßt.

12. Anwendung einer Vorichtung nach einem oder mehreren der Ansprüche 1 bis 11 für die Bestimmung der Aggregationsgeschwindigkeit der Erythrozyten in einer Blutprobe.

## Claims

1. A device for the investigation of flow phenomena comprising a capillary (1), which is transparent to light, an optical observation device (2) and a pump (25) for the production of a volumetric current of the liquid to be investigated in the capillary, characterized in that the pump (25) is a resiliently elastic tube (30) which is filled with an incompressible liquid and closed at one end, such tube being coupled with a deforming device (38, 39 and 40) with the aid of which said tube is subjected to a defined elastic flexure, under whose action the liquid-filled internal volume changes.

2. The device as claimed in claim 1, characterized in that the resiliently deforming tube (30) is circularly bent in the manner of a reversely connected tubular spring pressure gage and possesses an elliptical or flattened cross section.

3. The device as claimed in claim 1 or in claim 2, characterized in that the deforming device (38, 39 and 40) coupled with the elastically deformable spring tube (30) produces a cyclical flexure and return flexure of the spring tube (30).

4. The device as claimed in claim 3, characterized in that as a deforming device (38, 39 and 40) for the spring tube (30) use is made of a cam (38) driven by a geared motor (40).

5. The device as claimed in claim 4, characterized in that for changing the amplitude of the volumetric current the cam (38) is designed in the form of a conical eccentric body and is arranged in a sliding fashion on the driven shaft.

6. The device as claimed in claim 4 or in claim 5, characterized in that the shape of the cam (38) or of the eccentric body is such that between the two points of inflexion of the flexure movement a constant volumetric current is produced.

7. The device as claimed in any one or more of the preceding claims 1 through 6, characterized in that the frequency of oscillation of the spring tube (30) is able to be set from 0.1 to 3.0 Hz.

8. The device as claimed in any one or more of the claims 1 through 7, characterized in that the spring tube (30) is able to be coupled with a second cam (41), which is driven by its own geared motor (43) and causes a larger extension stroke of the spring tube (30) than the cam (38) producing the oscillating pump action.

9. The device as claimed in any one or more of the claims 1 through 8, characterized in that the capillary (1), which is transparent to light, possesses a flow cross section of 0.5 to 2 mm in width and 0.05 to 0.2 in height.

10. The device as claimed in any one or more of the claims 1 through 9, characterized in that the optical observation device (2) comprises an illuminating device with a light source (3) and a photometric microscope (7) for measuring the quantity of light shining through the capillary (1).

11. The device as claimed in any one or more of the claims 1 through 9, characterized in that the optical observation device comprises a light source (3) and one or more photo-diodes for measuring the quantity of light shining through the capillary.

12. The device as claimed in any one or more of the claims 1 through 11 for the determination of the aggregation rate of erythrocytes in a blood sample.

## Revendications

1. Dispositif pour l'examen d'écoulements comprenant un tube capillaire transparent (1), un dispositif d'observation optique (2) et une pompe (25) destinée à produire un débit volumique du liquide à examiner dans le tube capillaire,
caractérisé en ce que la pompe (25) est un tube (30) à déformation élastique fermé d'un côté et rempli d'un liquide incompressible, qui est couplé à un dispositif de déformation (38, 39, 40) et soumis à l'intervention de celui-ci à une flexion élastique définie sous l'effet de laquelle le volume intérieur rempli de liquide se modifie.

2. Dispositif suivant la revendication 1, caractérisé en ce que le tube à déformation élastique (30) est courbé en arc de cercle à la manière d'un manomètre à tube élastique monté à l'envers et présente une section transversale elliptique ou aplatie.

3. Dispositif suivant la revendication 1 ou 2, caractérisé en ce que le dispositif de déformation (38, 39, 40) couplé au tube à déformation élastique (30) produit une flexion et un retour de flexion cycliques du tube élastique (30).

4. Dispositif suivant la revendication 3, caractérisé en ce qu'un disque à came (38) entrainé par un motoréducteur (40) sert de dispositif de déformation (38, 39, 40) pour le tube élastique (30).

5. Dispositif suivant la revendication 4, caractérisé en ce que pour la modification de la grandeur du débit volumique, le disque à came (38) est conçu comme un corps excentrique conique et est disposé de manière à pouvoir coulisser sur l'axe de rotation entraîné.

6. Dispositif suivant la revendication 4 ou 5, caractérisé en ce que la forme du disque à came (38) ou du corps excentrique est prévue de telle sorte que, entre les deux points de renversement du mouvement de flexion, un débit volumique constant soit produit.

7. Dispositif suivant une ou plusieurs des revendications 1 à 6, caractérisé en ce que la fréquence d'oscillation du tube élastique (30) peut être réglée entre 0,1 et 3,0 Hz.

8. Dispositif suivant une ou plusieurs des revendications 1 à 7, caractérisé en ce que le tube élastique (30) peut être couplé à un deuxième disque à came (41) qui est entraîné par un motoréducteur propre (43) et qui imprime au tube élastique (30) une plus grande course d'extension que le disque à came (38) produisant l'effet de pompage oscillant.

9. Dispositif suivant une ou plusieurs des revendications 1 à 8, caractérisé en ce que le tube capillaire transparent (1) présente une section transversale d'écoulement plane de 0,5 à 2 mm de largeur et de 0,05 à 0,2 mm de hauteur.

10. Dispositif suivant une ou plusieurs des revendications 1 à 9, caractérisé en ce que le dispositif d'observation optique (2) comprend un dispositif d'éclairage pourvu d'une source lumineuse (3) et un photomètre-microscope (7) qui mesure la quantité de lumière qui traverse le tube capillaire (1).

11. Dispositif suivant une ou plusieurs des revendications 1 à 9, caractérisé en ce que le dispositif d'observation optique comprend un dispositif d'éclairage pourvu d'une source lumineuse (3) et une ou plusieurs photodiodes qui mesurent la quantité de lumière traversant le tube capillaire.

12. Utilisation d'un dispositif suivant l'une ou plusieurs des revendications 1 à 11, pour la détermination de la vitesse d'agrégation des érythrocytes dans une prise de sang.
